Europäisches Patentamt

(19) European Patent Office

Office européen des brevets

(11) EP 0 927 539 A1

(12) EUROPEAN PATENT APPLICATION

(43) Date of publication:
07.07.1999 Bulletin 1999/27

(51) Int. Cl.$^6$: A61B 8/00

(21) Application number: 98310298.9

(22) Date of filing: 16.12.1998

(84) Designated Contracting States:
AT BE CH CY DE DK ES FI FR GB GR IE IT LI LU
MC NL PT SE
Designated Extension States:
AL LT LV MK RO SI

(30) Priority: 16.12.1997 KR 9769307

(71) Applicant: MEDISON CO., LTD.
Kangwon-do 250-870 (KR)

(72) Inventor: Bae, Moo Ho
Nowon-gu, Seoul (KR)

(74) Representative:
Read, Matthew Charles et al
Venner Shipley & Co.
20 Little Britain
London EC1A 7DH (GB)

(54) Hand-held ultrasonic probe

(57) A free-hand ultrasonic probe for scanning a good quality of three-dimensional (3D) image with a comparatively simple structure is provided. The hand-held ultrasonic probe includes a transducer array (1) for emitting an ultrasonic beam corresponding to an imaging plane and receiving an ultrasonic echo from an object at a predetermined interval of time, a positioning sensor (30) for detecting a position shift amount of the transducer array at a predetermined interval of time, and a 3D image processor (20) for calculating a 3D image of the object using the received ultrasonic echoes, according to the position shift amount. Thereby a good quality of 3D images can be scanned by the probe even though a user moves the transducer array with an irregular angular velocity.

FIG. 2

## Description

The present invention relates to a three-dimensional ultrasonic probe, and more particularly, to a free-hand ultrasonic probe.

A three-dimensional ultrasonic probe is a device for scanning a volume image of an object using a Doppler effect of an ultrasonic echo. The three-dimensional ultrasonic probe is chiefly used for scanning a volume image of an object to which light can not reach like the inside of a human body. Recently, a free-hand ultrasonic probe of a comparatively simplified structure is widely used for scanning a three-dimensional (3D) image.

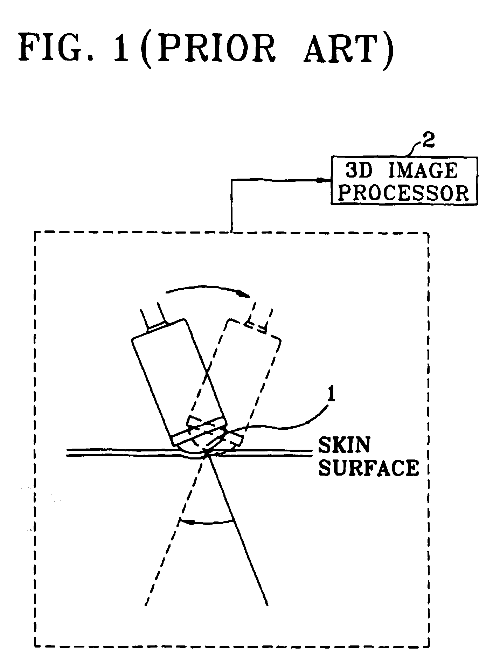

FIG. 1 shows a conventional free-hand ultrasonic probe. As shown in FIG. 1, a conventional free-hand ultrasonic probe comprises a transducer array 1 and a 3D image processor 2. The transducer array 1 is a one-dimensional array constituted with a plurality of transducer elements. The transducer array 1 emits an ultrasonic beam to form a fan-shaped imaging plane. When the emission of the ultrasonic beam is completed, the transducer array 1 receives an ultrasonic echo reflected from an object. The transducer array 1 alternately repeats the emission and reception operations at a predetermined interval of time. Also, the transducer array 1 converts the received ultrasonic echo into an electrical signal. The 3D image processor 2 receives the electrical signal from the transducer array 1, and then calculates an image of the object from the electrical signal. In the case that the 3D image processor 2 receives the ultrasonic echo one time, the image of the object obtained in the 3D image processor 2 is a slice image corresponding to the imaging plane. The image processor 2 integrates the slice images to calculate a 3D image of the object, in the case that the transducer array 1 moves to a one-direction regularly.

As described above, the free-hand ultrasonic probe of FIG. 1 scans a 3D image of the object using only essential components. However, to obtain a 3D image of an object, a user should move the transducer array 1 with a constant angular velocity, which causes much inconvenience to the user. In addition, since it is nearly impossible for the user to move the transducer array 1 with a constant angular velocity, the quality of a scanned 3D image is lowered.

To solve the above problems, it is an object of the present invention to provide a free-hand ultrasonic probe for scanning a 3D image with a comparatively simple structure, in which a positioning sensor detecting a position shift amount of a transducer array at a predetermined interval of time.

To accomplish the above object of the present invention, there is provided a free-hand ultrasonic probe comprising: a transducer array for emitting an ultrasonic beam corresponding to an imaging plane and receiving an ultrasonic echo from an object at a predetermined interval of time; a positioning sensor for detecting a position shift amount of the transducer array at a predetermined interval of time; and a three-dimensional (3D) image processor for calculating a 3D image of the object using the received ultrasonic echoes, according to the position shift amount.

The above object and other advantages of the present invention will become more apparent by describing the preferred embodiment thereof in more detail with reference to the accompanying drawings in which:

FIG. 1 shows a conventional free-hand ultrasonic probe;
FIG. 2 shows a free-hand ultrasonic probe according to a preferred embodiment of the present invention; and
FIG. 3 shows a free-hand ultrasonic probe according to another preferred embodiment of the present invention.

Preferred embodiments of the present invention will be described with reference to the accompanying drawings.

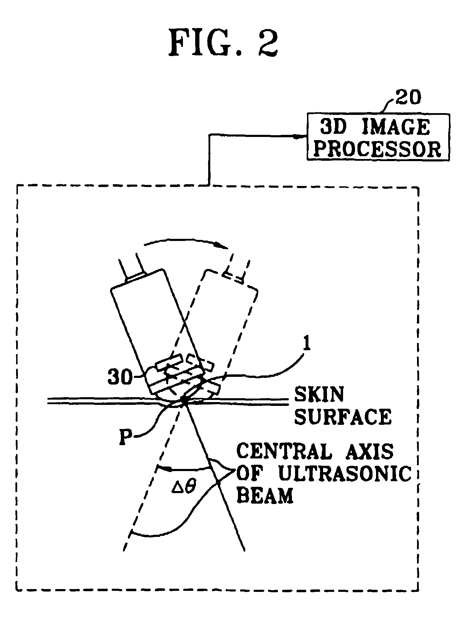

Referring to FIG. 2, a free-hand ultrasonic probe according to a preferred embodiment of the present invention comprises a transducer array 1, a 3D image processor 20 and a positioning sensor 30.

As can be seen from the reference numerals, the transducer array 1 performs the same as that of FIG. 1. That is, the transducer array 1 emits an ultrasonic beam corresponding to an imaging plane at a predetermined interval of time, and receives an ultrasonic echo reflected from an object. The transducer array 1 alternately repeats the emission and reception operations. Also, the transducer array 1 converts the received ultrasonic echo into an electrical signal, and outputs the converted result to the 3D image processor 20. The positioning sensor 30 detects a position shift amount $\Delta\theta$ of the transducer array 1 at a predetermined interval of time. The position shift amount $\Delta\theta$ is defined by $\theta(t_n) - \theta(t_{n-1})$. Here, $\theta(t_n)$ is a current position of the transducer array 1, and $\theta(t_{n-1})$ is the position of the just-previously detected transducer array 1. The 3D image processor 20 signal-processes the signal received from the transducer array 1 to obtain an image. In the case that the 3D image processor 20 receives the ultrasonic echo one time, the image of the object obtained in the 3D image processor 20 is a slice image corresponding to the imaging plane. The 3D image processor 20 obtains a position shift amount $\Delta\theta/\Delta t$ at a predetermined interval of time using the position shift amount $\Delta\theta$ detected by the positioning sensor 30, and then integrates the slice images depending upon the obtained values of $\Delta\theta/\Delta t$, by which a 3D image of an object is obtained.

As described above, in the case that a user moves the transducer array 1 in an arrow direction so that the central axis of the ultrasonic beam is swung at the state where a contact point P is fixed, a probe of FIG. 2 can scan a 3D image of an object which is located in the inside of the skin of the human body. Also, since the slice images are integrated according to the position shift amount $\Delta\theta$ at a predetermined interval of time, a good quality of 3D images can be scanned even though the transducer array 1 is swung with an irregular angular velocity.

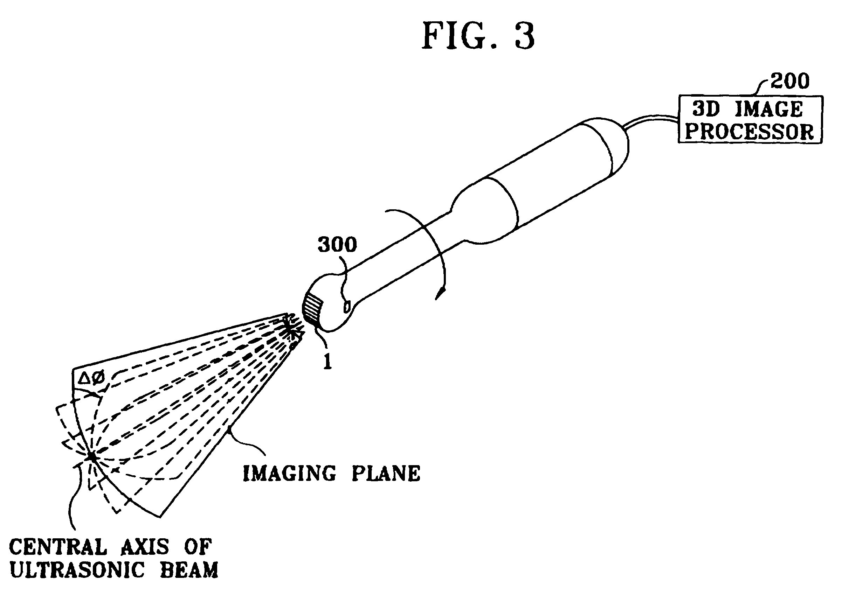

FIG. 3 shows a free-hand ultrasonic probe according to another preferred embodiment of the present invention. As shown in FIG. 3, a free-hand ultrasonic probe comprises a transducer array 1, a 3D image processor 200 and a positioning sensor 300.

As can be seen from the reference numeral, the transducer array 1 performs the same as that of FIG. 1 or 2. That is, the transducer array 1 emits an ultrasonic beam corresponding to an imaging plane at a predetermined interval of time, and receives an ultrasonic echo reflected from an object. The transducer array 1 alternately repeats the emission and reception operations. Also, the transducer array 1 converts the received ultrasonic echo into an electrical signal, and outputs the converted result to the 3D image processor 200. The positioning sensor 300 detects a position shift amount $\Delta\psi$ of the transducer array 1 at a predetermined interval of time. The position shift amount $\Delta\psi$ is defined by $\psi(t_n) - \psi(t_{n-1})$. Here, $\psi(t_n)$ is a current position of the transducer array 1, and $\psi(t_{n-1})$ is a position of the just-previously detected transducer array 1. The 3D image processor 200 signal-processes the signal received from the transducer array 1 to obtain an image. In the case that the 3D image processor 200 receives the ultrasonic echo one time, the image of the object obtained in the 3D image processor 200 is a slice image corresponding to the imaging plane. The 3D image processor 200 obtains a position shift amount $\Delta\psi/\Delta t$ at a predetermined interval of time using the position shift amount $\Delta\psi$ detected by the positioning sensor 300, and then integrates the slice images depending upon the obtained values of $\Delta\psi/\Delta t$, by which a 3D image of an object is obtained.

As described above, in the case that a user rotates the transducer array 1 in an arrow direction at the state where the central axis of the ultrasonic beam emitted is fixed, a probe of FIG. 3 can scan a 3D image of an object which is located in the inside of the skin of the human body. Also, since the slice images are integrated according to the position shift amount $\Delta\psi$ at a predetermined interval of time, a good quality of 3D images can be scanned even though a user rotates the transducer array 1 with an irregular angular velocity.

As described above, free-hand ultrasonic probe according to the present invention scans a 3D image of the object using only essential components. Also, a good quality of 3D images can be scanned by the probe even though a user moves the transducer array 1 with an irregular angular velocity.

Depending on the type of ultrasonic probe, the position sensor 30, 300 can be chosen from a range of known sensor types, including solid state accelerometers and inclinometers, which sense changes in angular position.

While certain embodiments of the invention have been specifically described herein, it will apparent that numerous modifications may be made thereto without departing from the spirit and scope of the invention.

## Claims

1. A free-hand ultrasonic probe comprising:

   a transducer array for emitting an ultrasonic beam corresponding to an imaging plane and receiving an ultrasonic echo from an object at a predetermined interval of time;
   a positioning sensor for detecting a position shift amount of the transducer array at said predetermined interval of time; and
   a three-dimensional (3D) image processor for calculating a 3D image of the object using the received ultrasonic echoes, according to the position shift amount.

2. The free-hand ultrasonic probe according to claim 1, wherein said 3D image processor obtains slice images from the ultrasonic echoes received at said predetermined interval of time, and then integrates the slice images according to the position shift amount.

3. The free-hand ultrasonic probe according to claim 1, wherein said positioning sensor detects a rotating angle of the transducer array at said predetermined interval of time in the case that the transducer array is rotated.

4. The free-hand ultrasonic probe according to claim 3, wherein said 3D image processor obtains the slice images from the ultrasonic echoes received at said predetermined interval of time, and then integrates the slice images according to the rotating angle.

5. The free-hand ultrasonic probe according to claim 1, wherein said positioning sensor detects a swing angle of the transducer array at said predetermined interval of time, in the case that the transducer array is swung at the state where the contact point is fixed.

6. The free-hand ultrasonic probe according to claim 5, wherein said processor obtains the slice images from the received ultrasonic echoes at said predetermined interval of time, and then integrates the slice images according to the swing angle.

7. A handheld ultrasonic probe for producing a three dimensional image of an object, comprising:

means (1) for producing a plurality of sectional images of the object;
means (30, 300) for providing information relating to the position of each of the sectional images in relation to the object; and
means (20, 200) for assembling the sectional images into a three dimensional image in dependence on the position information.

# FIG. 1 (PRIOR ART)

3D IMAGE PROCESSOR — 2

SKIN SURFACE — 1

# FIG. 2

# FIG. 3

200
3D IMAGE PROCESSOR

300

1

ΔØ

IMAGING PLANE

CENTRAL AXIS OF
ULTRASONIC BEAM

EP 0 927 539 A1

European Patent
Office

**EUROPEAN SEARCH REPORT**

Application Number

EP 98 31 0298

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 487 339 A (ADVANCED TECHNOLOGY LABORATORIES, INC.) 27 May 1992 * column 5, line 18 - line 31 * * column 7, line 16 - line 43 * * column 9, line 4 - line 55 * | 1-7 | A61B8/00 |
| X | EP 0 476 495 A (DORNIER MEDIZINTECHNIK) 25 March 1992 | 1-4,6,7 | |
| A | * column 2, line 47 - column 3, line 9 * * column 4, line 29 - column 6, line 4 * | 5 | |
| X | EP 0 749 722 A (HEWLETT-PACKARD CO.) 27 December 1996 | 1,3,5,6 | |
| A | * column 2, line 40 - column 3, line 26 * * column 4, line 8 - line 16 * * column 4, line 52 - column 5, line 7 * | 2,4,7 | |
| X | EP 0 432 771 A (ALOKA CO., LTD.) 19 June 1991 | 1,3 | |
| A | * column 4, line 3 - column 5, line 29 * * column 9, line 32 - column 10, line 2 * | 5,7 | TECHNICAL FIELDS SEARCHED (Int.Cl.6) A61B |

The present search report has been drawn up for all claims

| Place of search | Date of completion of the search | Examiner |
|---|---|---|
| THE HAGUE | 1 April 1999 | Rieb, K.D. |

EPO FORM 1503 03.82 (P04C01)

**ANNEX TO THE EUROPEAN SEARCH REPORT
ON EUROPEAN PATENT APPLICATION NO.**                EP 98 31 0298

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-1999

| Patent document cited in search report | | Publication date | Patent family member(s) | | Publication date |
|---|---|---|---|---|---|
| EP 487339 | A | 27-05-1992 | AT | 162701 T | 15-02-1998 |
| | | | DE | 69128827 D | 05-03-1998 |
| | | | DE | 69128827 T | 14-05-1998 |
| | | | EP | 0668051 A | 23-08-1995 |
| | | | JP | 4332544 A | 19-11-1992 |
| | | | US | 5529070 A | 25-06-1996 |
| | | | US | 5353354 A | 04-10-1994 |
| EP 476495 | A | 25-03-1992 | DE | 4029829 A | 02-04-1992 |
| | | | JP | 6022964 A | 01-02-1994 |
| EP 749722 | A | 27-12-1996 | JP | 9019431 A | 21-01-1997 |
| EP 432771 | A | 19-06-1991 | JP | 1944456 C | 23-06-1995 |
| | | | JP | 4122358 A | 22-04-1992 |
| | | | JP | 6075575 B | 28-09-1994 |
| | | | JP | 2009589 C | 02-02-1996 |
| | | | JP | 3184532 A | 12-08-1991 |
| | | | JP | 7038851 B | 01-05-1995 |
| | | | CA | 2032204 C | 14-03-1995 |
| | | | DE | 69027284 D | 11-07-1996 |
| | | | DE | 69027284 T | 05-12-1996 |
| | | | US | 5152294 A | 06-10-1992 |

EPO FORM P0459

For more details about this annex : see Official Journal of the European Patent Office, No. 12/82